(19) Europäisches Patentamt / European Patent Office / Office européen des brevets

(11) **EP 4 246 372 B1**

(12) **EUROPEAN PATENT SPECIFICATION**

(45) Date of publication and mention
of the grant of the patent:
**01.07.2026 Bulletin 2026/27**

(21) Application number: **22742287.0**

(22) Date of filing: **25.01.2022**

(51) International Patent Classification (IPC):
*G06V 10/24* (2022.01)    *G06V 10/20* (2022.01)
*G06V 40/16* (2022.01)

(52) Cooperative Patent Classification (CPC):
**G06V 40/166; G06V 10/243; G06V 10/255;
G06V 40/171**

(86) International application number:
**PCT/CN2022/073708**

(87) International publication number:
**WO 2022/156810 (28.07.2022 Gazette 2022/30)**

(54) **FACIAL SIZE DETERMINATION METHOD AND APPARATUS**

VERFAHREN UND VORRICHTUNG ZUR BESTIMMUNG DER GESICHTSGRÖSSE

PROCÉDÉ ET APPAREIL DE DÉTERMINATION DE TAILLE FACIALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.01.2021 CN 202110100112**

(43) Date of publication of application:
**20.09.2023 Bulletin 2023/38**

(73) Proprietor: **BMC (Tianjin) Medical Co., Ltd.
Tianjin 301700 (CN)**

(72) Inventors:
• **XU, Jian**
**Tianjin 301700 (CN)**
• **WANG, Huiquan**
**Tianjin 301700 (CN)**
• **ZHOU, Mingzhao**
**Tianjin 301700 (CN)**
• **HE, Long**
**Tianjin 301700 (CN)**
• **ZHUANG, Zhi**
**Tianjin 301700 (CN)**

(74) Representative: **Grünecker Patent- und
Rechtsanwälte
PartG mbB
Leopoldstraße 4
80802 München (DE)**

(56) References cited:
CN-A- 109 118 426    CN-A- 112 883 811
US-A1- 2012 169 868    US-A1- 2018 117 272
US-A1- 2018 299 704    US-B1- 9 536 301

**Description**

CROSS REFERENCE TO RELEVANT APPLICATIONS

The present disclosure claims the priority of the Chinese patent application filed on January 25th, 2021 before the Chinese Patent Office with the application number of 202110100112.4 and the title of "FACIAL SIZE DETERMINATION METHOD AND APPARATUS".

TECHNICAL FIELD

The present disclosure relates to the technical field of image processing, and particularly relates to a method and apparatus for determining a human-face size.

BACKGROUND

A method of non-invasive positive-pressure ventilation has been extensively used in Obstructive Sleep Apnea Syndrome (referred to for short as OSA), Chronic Obstructive Pulmonary Disease (referred to for short as COPD) and so on. No pipe is required to be inserted into the airway of the patient by surgery, but by using a blower, a pipeline and a patient interface device, a continuous pressure ventilation (CPAP) or varying pressure ventilation is delivered to the airway of the patient, for example, a double-level pressure varying with the respiratory cycle of the patient or an automatically regulated pressure ventilation varying with the monitored situation of the patient. Such a pressure-supporting therapy is usually also used in obstructive hypopnea, Upper Airway Resistance Syndrome (referred to for short as UARS), congestive heart failure and so on.

Non-invasive ventilation treatment device includes the interface device on the face of the patient. Such an interface device generally refers to a face mask that surrounds the nose and the mouth of the face of the patient and seals them. In the treatment, an external blower is the pressure supporting device, for example, a breathing machine, and the patient interface device connects the gas pressure supplied by the breathing machine and the airway of the patient, to send the respiratory air flow to the airway of the patient.

In order to adapt for different face sizes, in many cases the face masks are set to be different models, such as a large size, a middle size and a small size. In order to provide a more effective treatment, it is required to select the face mask that is suitable for the size of the face of the patient. When a face mask that does not match with the face size is selected, air leakage or other problem might happen, which affects the wearing comfortableness, and deteriorates the effect of the treatment. Therefore, it is very important to conveniently and quickly measure the face size of the patient, thereby selecting the face mask that is suitable for the face size of the patient. Regarding a patient having a malformed face, the face masks of the standard models cannot be used, and it is required to customize a dedicated face mask according to the contour of the face of the patient. In this case, it is required to conveniently and quickly acquire the 3D contour information of the face of the patient. US 2018/0299704 discloses a system and methods for creating fully custom products from scratch, wherein the system includes an image capture device for capturing image data and/or measurement data of a user; and a computer is communicatively coupled with image capture device and configured to construct an anatomic model of the user based on the capture image data and/or measurement data. US 2018/117272 A1 represents relevant prior art.

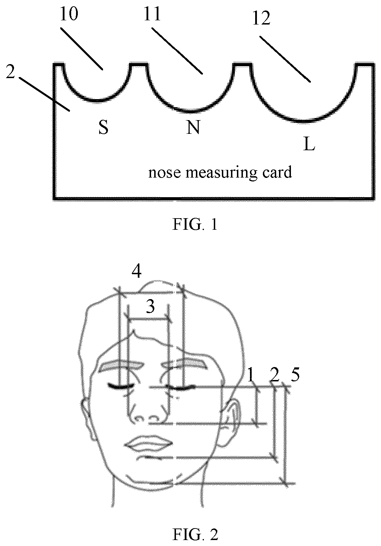

In the selection or personalized designing of medical face masks, a nose measuring card shown in FIG. 1 is usually used to measure the nose width. Most of the nose measuring cards for measuring the nose width in the related art are a card that can accommodate the nose of the patient. Regarding the nose measuring card shown in FIG. 1, the nose measuring card, according to the applicable models of face masks, is provided with three slots (10-12) of a small size (S), a middle size (M) and a large size (L), and corresponding model identifiers 2 are under the slots. In usage, the patient clips the slots of the nose measuring card on the nose, and, by using the model identifiers 2 on the nose measuring card, selects the suitable face-mask model.

In the related art, in the selection or personalized designing of the medical face masks, a measuring tool such as a scale ruler as shown in FIG. 2 may also be used to directly measure specific sizes of the face of the patient (for example, the 5 sizes in FIG. 2). According to those sizes in the human face, model selection or personalized designing of the face mask is performed.

The method of measuring the nose width by using the nose measuring card in the related art, in an aspect, can merely obtain an approximate range of the nose width of the patient, and cannot be used for the personalized customization of the face mask. In another aspect, when the model selection of the face mask is performed merely by using the nose width, the single factor is taken into consideration, and unsuitableness at the other positions such as the bridge of the nose and the chin might happen.

The method of directly measuring the face size of the patient by using a scale ruler in the related art has tedious data recording and troublesome operation, and the manual measurement easily has a high error.

In view of the above problems, no effective solutions have been proposed at present.

SUMMARY

The embodiments of the present disclosure provide a method and apparatus for determining a human-face size, which may accurately measure the human-face size of the to-be-measured target, to solve the technical problem in the related art of a low precision of measurement on human-face sizes.
The present invention is defined by the appended claims.
In the first aspect, an embodiment of the present disclosure provides a method for determining a human-face size, wherein the method includes:

collecting a first to-be-measured human-face image of a to-be-measured target, wherein the first to-be-measured human-face image includes a first to-be-measured human face of the to-be-measured target and a circular reference object;
acquiring a first value in pixel of the first to-be-measured human face, and a second value in pixel of the circular reference object;
in response to a circularity of the circular reference object being greater than a preset circularity threshold, acquiring an angle of inclination of the circular reference object; and
according to the first value in pixel, the second value in pixel, the angle of inclination and an actual size of the circular reference object, determining a first size of the first to-be-measured human face.

In the second aspect, an embodiment of the present disclosure further provides a method for determining a human-face size, wherein the method includes:

by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal;
acquiring a second value in pixel of the second to-be-measured human-face image, and a third value in pixel of the third to-be-measured human-face image; and
according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target.

In the third aspect, an embodiment of the present disclosure further provides a method for determining a human-face size, wherein the method comprises:

by using a third image collecting device, collecting a fourth to-be-measured human-face image of a to-be-measured target, wherein the third image collecting device comprises at least two cameras;
acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras;
according to the at least two incident angles of inclination and a camera distance between the at least two cameras, determining positions of the information collecting points; and
according to the positions of the information collecting points, determining a third size of a third to-be-measured human face in the fourth to-be-measured human-face image.

In the fourth aspect, an embodiment of the present disclosure further provides a method for determining a face-mask model, wherein the method includes:

by using the method for determining a human-face size according to any one of the first aspect, the second aspect and the third aspect, obtaining a target human-face size; and
according to a plurality of groups of preset face-mask reference values and the target human-face size, determining a face-mask model of the to-be-measured target, wherein the plurality of groups of preset face-mask reference values correspond to a plurality of face-mask models.

In the fifth aspect, an embodiment of the present disclosure further provides an apparatus for determining a human-face size, wherein the apparatus comprises:

a first collecting unit configured for collecting a first to-be-measured human-face image of a to-be-measured target, wherein the first to-be-measured human-face image includes a first to-be-measured human face of the to-be-measured target and a circular reference object;

a first acquiring unit configured for acquiring a first value in pixel of the first to-be-measured human face, and a second value in pixel of the circular reference object;

a second acquiring unit configured for, in response to a circularity of the circular reference object being greater than a preset circularity threshold, acquiring an angle of inclination of the circular reference object; and

a first determining unit configured for, according to the first value in pixel, the second value in pixel, the angle of inclination and an actual size of the circular reference object, determining a first size of the first to-be-measured human face.

In the sixth aspect, an embodiment of the present disclosure further provides an apparatus for determining a human-face size, wherein the apparatus includes:

a second collecting unit configured for, by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal;

a third acquiring unit configured for acquiring a second value in pixel of the second to-be-measured human-face image, and a third value in pixel of the third to-be-measured human-face image; and

a second determining unit configured for, according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target.

In the seventh aspect, an embodiment of the present disclosure further provides an apparatus for determining a human-face size, wherein the apparatus includes:

a third collecting unit configured for, by using a third image collecting device, collecting a fourth to-be-measured human-face image of a to-be-measured target, wherein the third image collecting device comprises at least two cameras;

a fourth acquiring unit configured for acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras;

a third determining unit configured for, according to the at least two incident angles of inclination and a camera distance between the at least two cameras, determining positions of the information collecting points; and

a fourth determining unit configured for, according to the positions of the information collecting points, determining a third size of a third to-be-measured human face in the fourth to-be-measured human-face image.

The embodiments of the present disclosure have the following advantages:

An embodiment of the present disclosure includes collecting a first to-be-measured human-face image of a to-be-measured target, wherein the first to-be-measured human-face image includes a first to-be-measured human face of the to-be-measured target and a circular reference object; acquiring a first value in pixel of the first to-be-measured human face, and a second value in pixel of the circular reference object; in response to a circularity of the circular reference object being greater than a preset circularity threshold, acquiring an angle of inclination of the circular reference object; and according to the first value in pixel, the second value in pixel, the angle of inclination and an actual size of the circular reference object, determining a first size of the first to-be-measured human face. By using the proportion relation between the actual size and the value in pixel in the first to-be-measured human-face image of the circular reference object, and according to the angle of inclination of the circular reference object, the first size of the first to-be-measured human face is determined, which simplifies the steps of the measuring operation in the related art, and increases the accuracy of the measurement on the human-face size.

Furthermore, another embodiment of the present disclosure includes by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal; acquiring a second value in pixel of the second to-be-measured human-face image, and a third value in pixel of the third to-be-measured human-face image; and according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target. By photographing the plurality of human-face images of the to-be-measured target at unequal image-collection distances, and subsequently using the image-collection-distance difference and the value in pixels of the different human-face images, the second size of the second to-be-measured human face is determined, which further increases the accuracy of the measurement on the human-face size.

Furthermore, another embodiment of the present disclosure includes by using a third image collecting device, collecting a fourth to-be-measured human-face image of a to-be-measured target, wherein the third image collecting device comprises at least two cameras; acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras; according to the at least two incident angles of inclination and a camera distance between the at least two cameras, determining positions of the information collecting points; and according to the positions of the information collecting points, determining a third size of a third to-be-measured human face in the fourth to-be-measured human-face image. That avoids manually measuring the human face by using a scale ruler, which simplifies the measuring operation. Moreover, by photographing the fourth to-be-measured human-face image by using a plurality of lenses, the third size of the third to-be-measured human face may be determined based on the camera distance between the cameras, which ensures the accuracy of the measurement on the human-face size.

The above description is merely a summary of the technical solutions of the present disclosure. In order to more clearly know the elements of the present disclosure to enable the implementation according to the contents of the description, and in order to make the above and other purposes, features and advantages of the present disclosure more apparent and understandable, the particular embodiments of the present disclosure are provided below.

## BRIEF DESCRIPTION OF THE DRAWINGS

In order to more clearly illustrate the technical solutions of the embodiments of the present disclosure, the figures that are required to describe the embodiments of the present disclosure will be briefly described below. Apparently, the figures that are described below are embodiments of the present disclosure, and a person skilled in the art can obtain other figures according to these figures without paying creative work.

FIG. 1 shows a schematic diagram of a nose measuring card in the related art;

FIG. 2 shows a schematic diagram of the direct measurement on a particular size of the face of a patient by using a measuring tool such as a scale ruler in the related art;

FIG. 3 shows a flow chart of the first embodiment of the method for determining a human-face size according to the present disclosure;

FIG. 4 shows a schematic diagram of a first to-be-measured human-face image at a standard angle according to the present disclosure;

FIG. 5 shows a schematic diagram of a first to-be-measured human-face image at a non-standard angle according to the present disclosure;

FIG. 6 shows a schematic diagram of a first to-be-measured human-face image including a circular reference object with a circularity greater than a preset threshold according to the present disclosure;

FIG. 7 shows a schematic diagram of a circular reference object with a circularity greater than a preset threshold according to the present disclosure;

FIG. 8 shows a flow chart of the second embodiment of the method for determining a human-face size according to the present disclosure;

FIG. 9 shows a schematic diagram of the collection of the to-be-measured human-face image by the second image collecting device at different image-collection distances according to the present disclosure;

FIG. 10 shows a flow chart of the third embodiment of the method for determining a human-face size according to the present disclosure;

FIG. 11 shows a schematic diagram of a method for acquiring incident angles of inclination of information collecting points based on a double-camera image collecting device according to the present disclosure;

FIG. 12 shows a schematic diagram of the distance between a plurality of information collecting points acquired based on a double-camera image collecting device according to the present disclosure;

FIG. 13 shows a schematic diagram of a method for determining a human-face size based on a three-camera image collecting device according to the present disclosure;

FIG. 14 shows a schematic diagram of a human-face model of a to-be-measured target according to an embodiment of the present disclosure;

FIG. 15 shows a structural block diagram of the first embodiment of the apparatus for determining a human-face size according to the present disclosure;

FIG. 16 shows a structural block diagram of the second embodiment of the apparatus for determining a human-face size according to the present disclosure;

FIG. 17 shows a structural block diagram of the third embodiment of the apparatus for determining a human-face size according to the present disclosure; and

FIG. 18 shows a structural block diagram of an electronic device according to the present disclosure.

DETAILED DESCRIPTION

The technical solutions of the embodiments of the present disclosure will be clearly and completely described below with reference to the drawings of the embodiments of the present disclosure. Apparently, the described embodiments are merely certain embodiments of the present disclosure, rather than all of the embodiments.

The first method embodiment

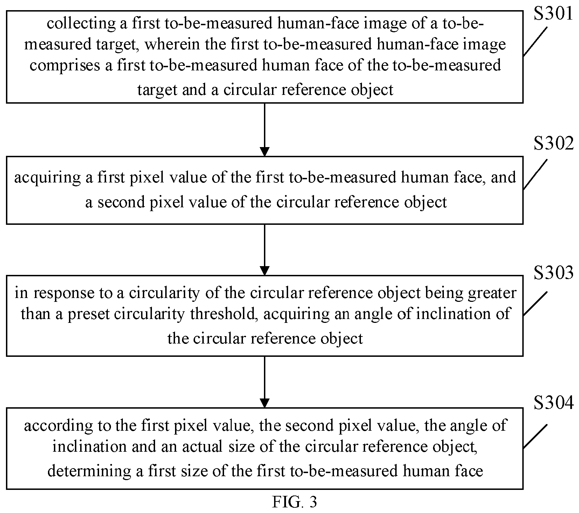

Referring to FIG. 3, FIG. 3 shows a flow chart of the first embodiment of the method for determining a human-face size according to the present disclosure. The method may particularly include:

S301: collecting a first to-be-measured human-face image of a to-be-measured target, wherein the first to-be-measured human-face image includes a first to-be-measured human face of the to-be-measured target and a circular reference object;

S302: acquiring a first value in pixel of the first to-be-measured human face, and a second value in pixel of the circular reference object;

S303: in response to a circularity of the circular reference object being greater than a preset circularity threshold, acquiring an angle of inclination of the circular reference object; and

S304: according to the first value in pixel, the second value in pixel, the angle of inclination and an actual size of the circular reference object, determining a first size of the first to-be-measured human face.

In the present embodiment, a first to-be-measured human-face image of a to-be-measured target is collected, wherein the first to-be-measured human-face image may be a photograph of the whole or a local part of a human body. The first to-be-measured human-face image includes the first to-be-measured human face that is required to be measured and a preset reference object, for example, a photograph of the full body, a photograph of the upper half body, a photograph of the head or the face, and so on, of a human body, which is not limited in any form in the present embodiment. The first image collecting device according to the present embodiment generally collects a planar image of the to-be-measured target, and the first image collecting device includes but is not limited to a photo camera, a video camera and other devices that have the function of image collection.

In the present embodiment, the circular reference object includes but is not limited to a coin, a circular cup cover, a circular disk and so on. Furthermore, the preset reference object further includes but is not limited to a circular scrip of a known size. In the present embodiment, the particular type and shape of the circular reference object are not limited in any form. The circularity according to the present embodiment refers to the difference between the radii of two concentric circles that include the same actual cross-sectional contour and have the lowest radius difference. The lower the value of the difference is, which indicates that the circle more tends to be a standard circle, and the higher the value of the difference is, which indicates that the circle deviates from a standard circle.

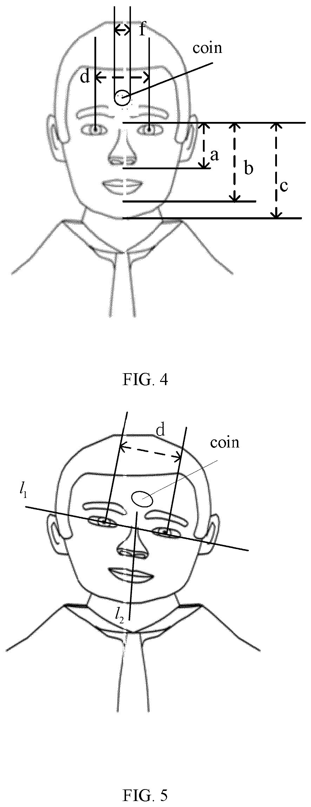

Furthermore, when the circularity of the circular reference object is less than or equal to the preset circularity threshold, it is deemed that, currently, the plane of the circular reference object and the plane where the image collecting device is located are parallel. Referring to FIG. 4, FIG. 4 shows the circular reference object that is placed on a forehead. In particular application scenes, the circular reference object includes but is not limited to a coin, a circular scrip and so on. In the case that the image-collection angle when the collecting device is collecting the first to-be-measured human-face image of the to-be-measured target is a standard angle, for example, the central axis of the lens of the camera is perpendicular to the plane of the circular reference object, or the camera is parallel to the plane of the circular reference object of the human face, then the circular reference object is presented as a standard circle in the first to-be-measured human-face image.

However, in the case that the image-collection angle when the collecting device is collecting the first to-be-measured human-face image of the to-be-measured target is a non-standard angle, the circularity of the circular reference object is greater than the preset circularity threshold, and therefore it is required to acquire the angle of inclination of the circular reference object. Referring to FIG. 5, FIG. 5 shows a schematic diagram of a first to-be-measured human-face image at a non-standard angle according to the present disclosure. When the image-collection angle is a non-standard angle, the circularity of the circular reference object increases, and the circular reference object is presented as an ellipse. In this case, when the first size of the first human face is determined according to the value in pixel and the actual size of the circular reference object, a measurement deviation emerges.

When the circularity of the circular reference object is greater than a preset circularity threshold, it is determined that the first to-be-measured human face including the circular reference object is photographed at a non-standard angle, and accordingly it is deemed that the first to-be-measured human-face image is a projection on the photograph/negative film. Therefore, it is required to determine the angle of inclination of the circular reference object, to rectify the deviation of the angle of inclination. Therefore, when the circularity of the circular reference object in the first to-be-measured human-face image is greater than the preset circularity threshold, it is required to determine the angle of inclination of the circular reference object, and, after the angle of inclination of the circular reference object is determined, the first size of the first to-be-measured human face according to the angle of inclination is subsequently determined.

In other examples of the present embodiment, on the above basis, the coin adhered to the forehead of the to-be-measured target is replaced by a credit card, which, certainly, may also be a circular non-standard item whose size may be easily measured (including but not limited to a circular scrip). The implementations and the calculation processes of the other examples of the present embodiment are the same as the principle when a coin is used as the preset reference object, and are not discussed further herein.

It should be noted that the present embodiment comprises collecting a first to-be-measured human-face image of a to-be-measured target, wherein the first to-be-measured human-face image includes a first to-be-measured human face of the to-be-measured target and a circular reference object; acquiring a first value in pixel of the first to-be-measured human face, and a second value in pixel of the circular reference object; if a circularity of the circular reference object is greater than a preset circularity threshold, acquiring an angle of inclination of the circular reference object; and according to the first value in pixel, the second value in pixel, the angle of inclination and an actual size of the circular reference object, determining a first size of the first to-be-measured human face. By using the proportion relation between the actual size and the value in pixel in the first to-be-measured human-face image of the circular reference object, and according to the angle of inclination of the circular reference object, the first size of the first to-be-measured human face is determined, which simplifies the steps of the measuring operation in the related art, and increases the accuracy of the measurement on the human-face size.

Optionally, the present embodiment further comprises but is not limited to: if the circularity is less than or equal to the preset circularity threshold, according to the first value in pixel, the second value in pixel and the actual size, determining the first size.

Particularly, in an example, referring to FIG. 4, FIG. 4 shows a schematic diagram of the first to-be-measured human-face image at a standard angle according to the present disclosure, in which a standardized coin is used as the preset reference object. When the first to-be-measured human-face image of the to-be-measured target is being collected at the standard angle, the coin is pressed at the center of the forehead of the human face of the to-be-measured target. Because the coin is closely adhered to the center of the forehead, it can be understood that the coin and the human face are in the same plane. As a known condition, the diameter of a 1 yuan coin is 22.25mm. When the image collection is performed at a standard angle, it may be deemed that the camera is parallel to the plane where the human face of the to-be-measured target is located. In this case, the value in pixel f of the diameter of the coin in the first to-be-measured human-face image is measured. Accordingly, according to the proportion relation between the actual size and the value in pixel in the first to-be-measured human-face image, it may be obtained that:

$$\frac{22.25}{f} = \frac{A}{a} = \frac{B}{b} = \frac{C}{c} = \frac{D}{d} \qquad (3)$$

By using the above formula, the actual sizes of the particular positions in the first to-be-measured human face may be obtained as follows:

$$A = \frac{22.25a}{f}, B = \frac{22.25b}{f}, C = \frac{22.25c}{f}, D = \frac{22.25d}{f} \qquad (4)$$

By using the above example, in response to the circularity of the circular reference object being less than the preset circularity threshold, the first size of the first to-be-measured human face may be determined according to the first value in pixel, the second value in pixel and the actual size of the circular reference object, which increases the precision of the acquired human-face size.

Optionally, in the present embodiment, in response to the circularity being greater than the preset circularity threshold, the step of determining the angle of inclination of the circular reference object includes but is not limited to: acquiring a standard major axis of the circular reference object, wherein the standard major axis refers to, in response to the circularity being greater than the preset circularity threshold, a longest diameter in the circular reference object; acquiring a first major axis in a first direction and a second major axis in a second direction in the circular reference object, wherein the first direction refers to a straight line where a connecting line between centers of two eyes in the first to-be-measured human-face image is located, and the second direction refers to a straight line where a connecting line between an eyebrow center and a nasal tip in the first to-be-measured human-face image is located; according to the standard major axis and the first major axis, determining a horizontal angle of inclination of the circular reference object; and according to the standard major axis and the second major axis, determining a vertical angle of inclination of the circular reference object.

Particularly, referring to FIG. 5, FIG. 5 shows a schematic diagram of the first to-be-measured human-face image when the preset reference object is a coin. That is because, when the first to-be-measured human-face image of the to-be-measured target is being collected, the orientation of the first image collecting device and the human face of the to-be-measured target have a certain angle of inclination therebetween. In this case, the photograph may be considered as a projection of the contour of the first to-be-measured human face on the paper plane (the plane where the negative film or the roll film is located). In the projection direction, it may be deemed that the above angle of inclination is formed by a horizontal angle of inclination $\alpha$ and a vertical angle of inclination $\beta$. As shown in FIG. 5, the connecting line between the centers of the two eyes in the face plane of the to-be-measured target is defined as $l_1$, and the connecting line of the eyebrow center, the nasal tip and the philtrum (the depression in the middle over the upper lip) is defined as $l_2$. In the space coordinate system, the horizontal angle of inclination $\alpha$ refers to the included angle between the $l_1$ in the face plane and its projection in the paper plane, and the vertical angle of inclination $\beta$ refers to the included angle between the $l_2$ in the face plane and its projection in the paper plane.

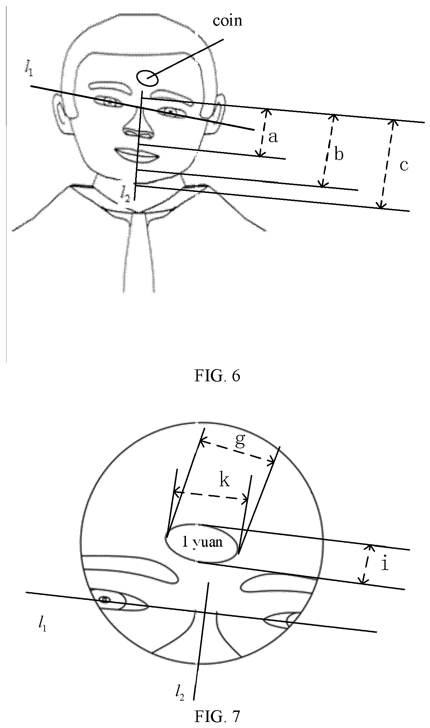

Referring to FIG. 6 and FIG. 7, FIG. 6 shows a schematic diagram of a first to-be-measured human-face image including a circular reference object with a circularity greater than a preset threshold according to the present disclosure, and FIG. 7 shows a schematic diagram of a circular reference object according to the present disclosure. The value in pixel g of the standard major axis (the diameter in the longest direction of the circular reference object) of the circular reference object in the photograph is identified, the value in pixel k of the circular reference object in the direction $l_1$ in the first to-be-measured human-face image is identified, and the value in pixel i of the circular reference object in the direction $l_2$ in the first to-be-measured human-face image is identified. It is easily obtained that the value in pixel g of the standard major axis of the circular reference object is equal to the value in pixel of the diameter when there is no angle of inclination (the camera and the face plane are parallel). It can be understood that the value in pixel k is equal to the projection of the value in pixel g of the major axis in the direction $l_1$, and the value in pixel i is equal to the projection of the value in pixel g of the major axis in the direction $l_2$. Accordingly, the horizontal angle of inclination and the vertical angle of inclination may be determined by using the following formulas:

$$\cos\alpha = \frac{k}{g}, \cos\beta = \frac{i}{g} \tag{5}$$

$$\alpha = \arccos\frac{k}{g}, \beta = \arccos\frac{i}{g} \tag{6}$$

Optionally, in the present embodiment, the step of, according to the first value in pixel, the second value in pixel and the actual size of the preset reference object, determining the first size of the first to-be-measured human face includes but is not limited to: according to the first value in pixel, the second value in pixel, the horizontal angle of inclination, the vertical angle of inclination and the actual size, determining the first size.

Particularly, when the circular reference object is a 1 yuan coin, the diameter of the 1 yuan coin is 22.25mm. In FIG. 4, according to the proportion relation between the actual size and the value in pixel of the 1 yuan coin, it may be easily obtained that the projection distance in $l_1$ between the centers of the two eyes is $\dfrac{22.25}{g}d$ , and accordingly the true distance in the space between the centers of the two eyes is:

$$D = \frac{22.25}{g}d \div \cos\alpha \tag{7}$$

By substituting the $\cos\alpha = \dfrac{k}{g}$ in the formula (5) into formula (7), it is obtained that:

$$D = \frac{22.25}{g}d \div \frac{k}{g} = \frac{22.25d}{k} \tag{8}$$

Likewise, based on FIG. 5, it can be obtained that:

$$A = \frac{22.25a}{i}, B = \frac{22.25b}{i}, C = \frac{22.25c}{i} \tag{9}$$

Optionally, in the present embodiment, before the step of collecting the first to-be-measured human-face image of the to-be-measured target, the method further includes but is not limited to: on an image previewing interface of the first image collecting device for collecting the first to-be-measured human-face image, exhibiting an auxiliary line, wherein the auxiliary line is used to indicate the spatial position and the image-collection angle of the first image collecting device.

Particularly, in order to effectively reduce the error generated in the collection of the human-face image of the to-be-measured target at an image-collection angle as a non-standard angle, a photographing assisting means may be provided in the first image collecting device. For example, when the human-face image of the to-be-measured target is being collected, an auxiliary line is exhibited in a preview frame of the lens image, to guide the user to adjust the position of the camera, to reduce the horizontal angle of inclination and the vertical angle of inclination. The auxiliary line may be a horizontal line, to guide the user to, in photographing, make his eyes to flush with the horizontal line, and may also be a rectangular auxiliary line or circular auxiliary line that has the similar function, so as to place the preset reference object within the area of the auxiliary line and so on.

Optionally, in the present embodiment, the circular reference object includes but is not limited to an iris in the first to-be-measured human-face image.

The present embodiment includes collecting a first to-be-measured human-face image of a to-be-measured target, wherein the first to-be-measured human-face image includes a first to-be-measured human face of the to-be-measured target and a circular reference object; acquiring a first value in pixel of the first to-be-measured human face, and a second value in pixel of the circular reference object; in response to a circularity of the circular reference object being greater than a preset circularity threshold, acquiring an angle of inclination of the circular reference object; and according to the first value in pixel, the second value in pixel, the angle of inclination and an actual size of the circular reference object, determining a first size of the first to-be-measured human face. By using the proportion relation between the actual size and the value in pixel in the first to-be-measured human-face image of the circular reference object, and according to the angle of inclination of the circular reference object, the first size of the first to-be-measured human face is determined, which simplifies the steps of the measuring operation in the related art, and increases the accuracy of the measurement on the human-face size.

The second method embodiment

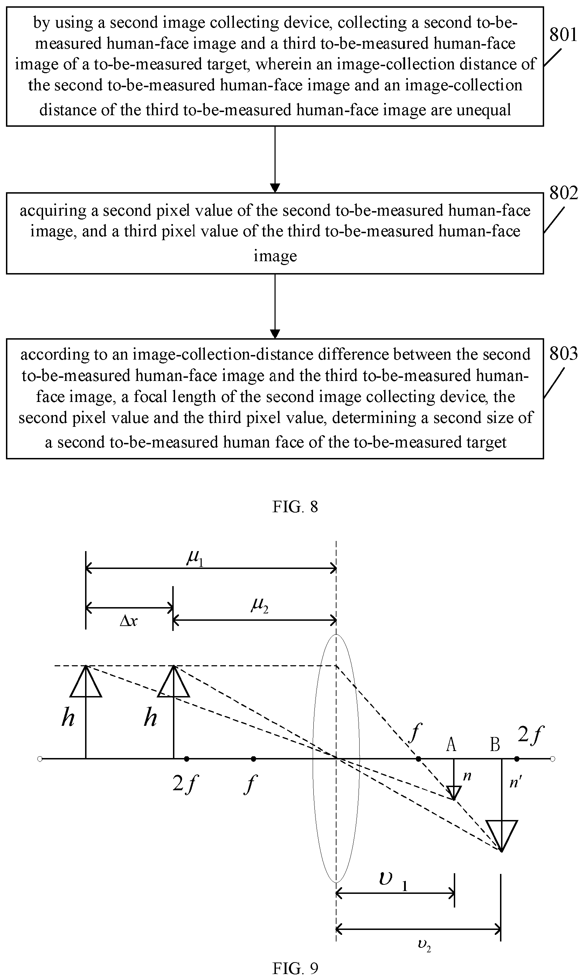

Referring to FIG. 8, FIG. 8 shows a flow chart of the second embodiment of the method for determining a human-face size according to the present disclosure. The method may particularly include:

Step 801: by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal;

Step 802: acquiring a second value in pixel of the second to-be-measured human-face image, and a third value in pixel of the third to-be-measured human-face image; and

Step 803: according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target.

In the present embodiment, the collected second to-be-measured human-face image and third to-be-measured human-face image of the to-be-measured target include but are not limited to a photograph of the whole or a local part of a human body. The second to-be-measured human-face image or the third to-be-measured human-face image includes the second to-be-measured human face that is required to be measured, for example, a photograph of the full body, a photograph of the upper half body, a photograph of the head or the face, and so on, of a human body. In the present embodiment, the particular photograph type is not limited in any form.

The second image collecting device according to the present embodiment generally collects a planar image of the to-be-measured target, and the second image collecting device includes but is not limited to a photo camera, a video camera and other devices that have the function of image collection. It should be noted that, in the process of the second image collecting device collecting the second to-be-measured human-face image and the third to-be-measured human-face image of the to-be-measured target, the image-collection distances of the collection of the second to-be-measured human-face image and the collection of the third to-be-measured human-face image are unequal, but the image-collection angles are equal. In order to maintain the accuracy of the acquired second human-face size, it is required to maintain the camera and the human-face plane of the second to-be-measured human face to be parallel.

Furthermore, optionally, the present embodiment includes but is not limited to the second to-be-measured human-face image and the third to-be-measured human-face image that are obtained by performing twice collection at unequal distances by the second image collecting device, and may also include at least two human-face images that are obtained by performing at least twice collection at unequal distances by the second image collecting device, for example, the second to-be-measured human-face image, the third to-be-measured human-face image and another to-be-measured human-face image that are obtained by collection at three unequal distances. By collecting the plurality of to-be-measured human-face images, the accuracy of the second to-be-measured human face that is actually measured is increased, thereby reducing the error.

Referring to FIG. 9, FIG. 9 shows a schematic diagram of the collection of the to-be-measured human-face image by the second image collecting device at different image-collection distances according to the present disclosure. The camera in the image collecting device is essentially a convex lens. The point where parallel light rays converge after passing through the convex lens is referred to as the focal point, and the distance from the focal point to the center of the convex lens is referred to as the focal length, which is expressed by the letter f. The focal length f of a camera is decided by the curvature of the convex lens itself, and convex lenses of different curvatures have different focal lengths f. The focal lengths f of the cameras of commonly used video cameras or photo cameras may be obtained from the specification of the product or from the manufacturer, or may be preset. In the image collection, the distance from the to-be-measured target to the center of the convex lens of the camera is the image-collection distance $\mu$, and the distance from the roll film (the imaging position) to the center of the convex lens of the camera is the image distance v. When the second image collecting device is operating, the object is beyond 2 times the focal length ($\mu > 2f$), and the roll film (the imaging position) is between 1 times the focal length and 2 times the focal length ($f < \upsilon < 2f$), to form an upside-down and reduced image.

In the example in FIG. 9, when a specified object of the size h in the second to-be-measured human face of the to-be-measured target is at the position 1 whose image-collection distance is $\mu_1$, the size of the image at the point A is $n$, in which case the image distance is $v_1$. When it is at the position 2 whose image-collection distance is $\mu_2$, the size of the image at the point B is $n'$, in which case the image distance is $v_2$. According to the principle of convex-lens imaging, it may be obtained that:

$$\frac{1}{\mu_1} + \frac{1}{\upsilon_1} = \frac{1}{f} \tag{10}$$

$$\frac{1}{\mu_2} + \frac{1}{\upsilon_2} = \frac{1}{f} \tag{11}$$

In FIG. 9, according to the principle of similitude of right triangles, it may be obtained that:

$$\frac{\mu_1}{\upsilon_1} = \frac{h}{n} \qquad (12)$$

$$\frac{\mu_2}{\upsilon_2} = \frac{h}{n'} \qquad (13)$$

In most cases, the measurement on the image-collection distances $\mu_1$ and $\mu_2$ is difficult. If it is defined that the difference between the two image-collection distances is:

$$\triangle X = \mu_1 - \mu_2 \qquad (14)$$

Then, by simultaneously solving the formulas (10)-(14), the object size may be obtained:

$$h = \frac{\triangle X}{f} \cdot \frac{nn'}{n'-n}, \quad \mu_2 = \frac{\upsilon_2 \triangle X}{f} \cdot \frac{n}{n'-n} \qquad (15)$$

It can be known from the formula (15) that it is merely required to measure the difference $\Delta X$ between the image-collection distances, and the sizes $n$ and $n'$ of the same object in the photographs collected in two different images, and the true size of the specified object in the second to-be-measured human face may be estimated. It can be understood that, when the object size h is the sizes of multiple features in the face surface of the patient, the true sizes of the multiple features may be individually estimated.

In practical application scenes, when there is not a high requirement on the size precision, the image distance $v_2$ is approximately equal to the focal length $f(\upsilon_2 \approx f)$. The image-collection distance from the specified object to the camera may be estimated as follows:

$$\mu_2 = \frac{n}{n'-n} \triangle X \qquad (16)$$

If the specified object is a certain tiny feature of the to-be-measured target, the distance from the tiny feature to the photo camera may be estimated.

Likewise, the image-collection distances from each of the tiny features (for example, point features and line features) of the face of the to-be-measured target to the photo camera may be estimated. By performing data processing to the image-collection distances from each of the point features and the line features of the face of the to-be-measured target to the camera, a 3D contour diagram of the face of the to-be-measured target may be obtained, to obtain the second size of the second to-be-measured human face of the to-be-measured target.

Optionally, in the present embodiment, the step of, by using the second image collecting device, collecting the second to-be-measured human-face image and the third to-be-measured human-face image of the to-be-measured target incudes but is not limited to: according to a first position sensor in the second image collecting device, and a second position sensor in the to-be-measured target, determining the image-collection-distance difference.

Particularly, the first position sensor and the second position sensor are provided in the second image collecting device and the to-be-measured target respectively, a first image-collection distance corresponding to the second to-be-measured human-face image and a second image-collection distance corresponding to the third to-be-measured human-face image are determined by using the first position sensor and the second position sensor, and subsequently the difference between the first image-collection distance and the second image-collection distance is acquired.

It should be noted that the first position sensor and the second position sensor according to the present embodiment include but are not limited to an alignment sensor, an infrared sensor and so on. When the first position sensor and the second position sensor are alignment sensors, by using the first position sensor and the second position sensor, the real-time positions of the image collecting device and the to-be-measured target may be determined respectively, thereby determining the image-collection-distance difference. Moreover, when the first position sensor and the second position sensor are infrared sensors, the distance between the first position sensor and the second position sensor may be acquired, thereby determining the image-collection-distance difference. In the present embodiment, the particular types of the first position sensor and the second position sensor are not limited in any form.

Optionally, in the present embodiment, the step of, by using the second image collecting device, collecting the second to-be-measured human-face image and the third to-be-measured human-face image of the to-be-measured target includes but is not limited to: after collecting the second to-be-measured human-face image by using the second image collecting device, controlling the second image collecting device to move by a first distance, wherein the first distance is equal to the image-collection-distance difference; or after collecting the second to-be-measured human-face image by using the second image collecting device, controlling the to-be-measured target to move by the first distance.

Particularly, in the present embodiment, the image-collection distance may be changed in two modes. The first mode is to maintain the position of the to-be-measured target unchanged, and move the second image collecting device relative to the to-be-measured target by the first distance. The second mode is to maintain the position of the second image collecting device unchanged, and control the to-be-measured target to be moved relative to the second image collecting device by the first distance. It should be noted that, after the second image collecting device or the to-be-measured target is moved by the first distance, it is required to maintain the camera of the second image collecting device and the human-face plane of the to-be-measured target to be parallel.

The present embodiment includes by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal; acquiring a second value in pixel of the second to-be-measured human-face image, and a third value in pixel of the third to-be-measured human-face image; and according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target. By photographing the plurality of human-face images of the to-be-measured target at unequal image-collection distances, and subsequently using the image-collection-distance difference and the value in pixels of the different human-face images, the second size of the second to-be-measured human face is determined, which further increases the accuracy of the measurement on the human-face size.

The third method embodiment

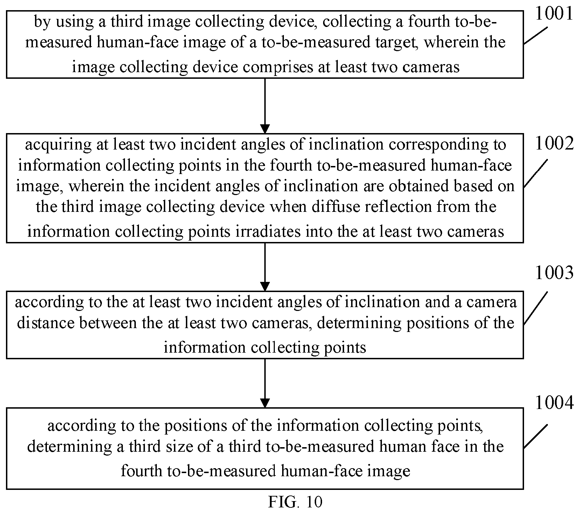

Referring to FIG. 10, FIG. 10 shows a flow chart of the third embodiment of the method for determining a human-face size according to the present disclosure. The method may particularly include:

Step 1001: by using a third image collecting device, collecting a fourth to-be-measured human-face image of a to-be-measured target, wherein the image collecting device includes at least two cameras;
Step 1002: acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras;
Step 1003: according to the at least two incident angles of inclination and a camera distance between the at least two cameras, determining positions of the information collecting points; and
Step 1004: according to the positions of the information collecting points, determining a third size of a third to-be-measured human face in the fourth to-be-measured human-face image.

In the present embodiment, the fourth to-be-measured human-face image includes but is not limited to a photograph of the whole or a local part of a human body. The fourth to-be-measured human-face image includes the third to-be-measured human face that is required to be measured, for example, a photograph of the full body, a photograph of the upper half body, a photograph of the head or the face, and so on, of a human body. In the present embodiment, the particular photograph type is not limited in any form.

The third image collecting device according to the present embodiment includes but is not limited to a photo camera, a video camera and other devices that have the function of image collection. The third image collecting device includes at least two cameras, and the cameras have the function of data processing, and may acquire the incident angles of inclination of light rays. Preferably, the at least two cameras in the third image collecting device are arranged according to a predetermined layout. For example, when the image collecting device is a double-camera image collecting device, the straight line where the connecting line between the two cameras is located is a straight line in the vertical or horizontal direction, and is parallel to the plane where the human face of the to-be-measured target is located. Moreover, when the image collecting device is an image collecting device having three or more cameras, all of the three or more cameras are provided in the same plane, and are arranged at the positions of the vertexes of a polygon, wherein the quantity of the sides of the polygon is equal to the quantity of the cameras. For example, in an image collecting device having three cameras, the three cameras are arranged at the positions of the vertexes of a triangle. Four cameras are arranged at the positions of the vertexes of a square.

The cameras in the third image collecting device according to the present embodiment have the function of data processing, and may acquire the incident angles of inclination of light rays. Practically, by using the position layout of the cameras in the third image collecting device, the distance between the plurality of cameras may be acquired, and, subsequently, by using the distance between the plurality of cameras and the information collecting points in the human face of the to-be-measured target, the positions of the information collecting points may be determined.

In particular application scenes, a plurality of information collecting points are provided in advance in the human face of the to-be-measured target, and, by acquiring the positions of the plurality of information collecting points, a three-dimensional 3D model of the human face of the to-be-measured target is established. The quantity and the particular positions of the information collecting points may be set according to practical experience. For example, the information collecting points are configured according to the requirements of breathing masks in practical applications, for example, the two-eye distance, the distance from the nasal tip to the human eyes, the bridge height, and so on, in the human face, which is not limited in any form in the present embodiment.

Optionally, in the present embodiment, the third image collecting device includes a first camera and a second camera, and the information collecting points include a first end point and a second end point of a preset connecting line of the fourth to-be-measured human-face image; and the step of acquiring the at least two incident angles of inclination corresponding to the information collecting points in the fourth to-be-measured human-face image includes but is not limited to: acquiring an incident angle of inclination corresponding to the first end point and an incident angle of inclination corresponding to the second end point, wherein the preset connecting line is parallel to a connecting line between the first camera and the second camera.

Particularly, when the third image collecting device has merely two cameras, the acquired distances from the information collecting points to the first camera and to the second camera are actually relative distances. The connecting line formed by the first camera and the second camera is parallel to the preset connecting line, and the information collecting points in the preset connecting line are located in the plane formed by the first camera, the second camera, the first end point and the second end point. By acquiring the incident angles of inclination corresponding to the at least two information collecting points in the preset connecting line, i.e., the first end point and the second end point, by using the double-camera third image collecting device, the relative positions of the first end point and the second end point are determined.

Optionally, in the present embodiment, the step of acquiring the incident angle of inclination corresponding to the first end point and the incident angle of inclination corresponding to the second end point includes but is not limited to: acquiring a first incident angle of inclination of irradiation from the first end point into the first camera, and a second incident angle of inclination of irradiation from the first end point into the second camera; and acquiring a third incident angle of inclination of irradiation from the second end point into the first camera, and a fourth incident angle of inclination of irradiation from the second end point into the second camera.

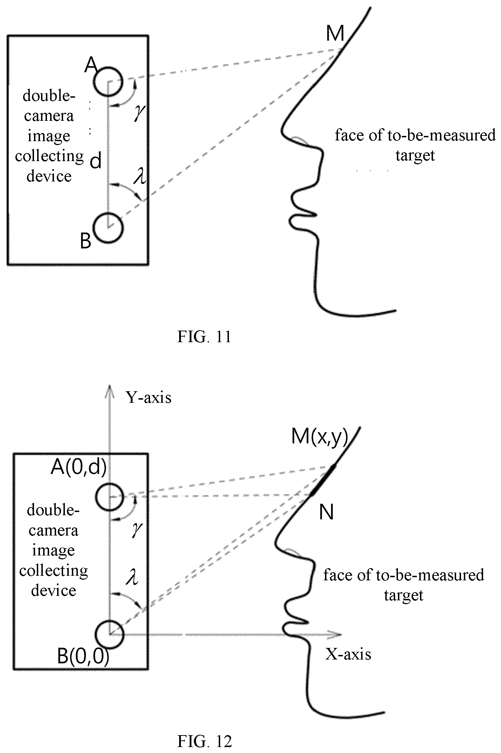

Particularly, referring to FIG. 11, FIG. 11 shows a schematic diagram of a method for acquiring incident angles of inclination of information collecting points based on a double-camera image collecting device according to the present disclosure. By using a double-lens processing system, the size information of the to-be-measured target is obtained. The double-camera image collecting device includes a camera A and a camera B, and has the function of data processing. The distance between the camera A and the camera B is used as a known condition, and is expressed by the letter d. The camera A and the camera B can capture light rays, and sense the incident angles of inclination of the light rays. The first end point M of the preset connecting line in the face of the to-be-measured target is taken, and the light rays that it diffusively reflects individually enter the camera A and the camera B. The camera A captures the light ray MA from the first end point M, and calculates to obtain its incident angle of inclination $\gamma$. The camera B captures the light ray MB from the first end point M, and calculates to obtain its incident angle of inclination $\lambda$. Based on the same logic and calculation mode as those of the first end point M, the incident angle of inclination of the second end point of the preset connecting line may be obtained similarly, which is not discussed further herein.

Optionally, in the present embodiment, the step of, according to the at least two incident angles of inclination and the camera distance between the at least two cameras, determining the positions of the information collecting points includes but is not limited to: according to the first incident angle of inclination, determining a first distance between the first end point and the first camera, and according to the first incident angle of inclination, determining a second distance between the first end point and the second camera; according to the second incident angle of inclination, determining a third distance between the second end point and the first camera, and according to the second incident angle of inclination, determining a fourth distance between the second end point and the second camera; based on a straight line where the first camera and the second camera are located, establishing a plane coordinate system; based on the plane coordinate system, according to the first distance, the second distance, the first incident angle of inclination, the second incident angle of inclination and the camera distance, determining a plane-coordinate position of the first end point; and based on the plane coordinate system, according to the third distance, the fourth distance, the third incident angle of inclination, the fourth incident angle of inclination and the camera distance, determining a plane-coordinate position of the second end point.

Particularly, referring to FIG. 12, FIG. 12 shows a schematic diagram of the distance between the first end point and the second end point acquired based on a double-camera image collecting device according to the present disclosure. The example in FIG. 11 gives the method for acquiring the incident angles of inclination of the first end point M and the second end point N. In FIG. 12, taking the first end point M as an example, a triangle MAB is established in the plane, and, accordingly, by using the triangle sine law, it may be obtained that:

$$|MA| = \frac{d \sin \lambda}{\sin \gamma \cos \lambda + \cos \gamma \sin \lambda} \qquad (17)$$

$$|MB| = \frac{d \sin \gamma}{\sin \gamma \cos \lambda + \cos \gamma \sin \lambda} \qquad (18)$$

In order to further determine the position of the first end point M, the present disclosure provides the following preferable logical-calculation method. By using the camera B as the origin of coordinates, using the direction of the connecting line between the camera B and the camera A as the Y-axis direction, and using the normal direction of the connecting line between the camera B and the camera A as the X-axis direction, a rectangular plane coordinate system XOY is established (FIG. 10). It can be understood that the coordinate of the point of the camera B is (0,0), and the coordinate of the point of the camera A is (0,d). It is set that the coordinate of the information collecting point M is (x,y), and accordingly the following vector relation may be obtained:

$$BA = (0,d); BM = (x,y); AB = (0,-d); AM = (x, y-d) \qquad (19)$$

$$\cos \lambda = \frac{BA \cdot BM}{|BA| \cdot |BM|} = \frac{dy}{d \cdot \sqrt{x^2 + y^2}} = \frac{y}{\sqrt{x^2 + y^2}} \qquad (20)$$

$$\cos \gamma = \frac{AB \cdot AM}{|AB| \cdot |AM|} = \frac{-dy + d^2}{d \cdot \sqrt{x^2 + (y-d)^2}} = \frac{-y+d}{\sqrt{x^2 + (y-d)^2}} \qquad (21)$$

By simultaneously solving the formulas (20)-(21), it may be obtained that:

$$x = \frac{d \tan \gamma \tan \lambda}{\tan \gamma - \tan \lambda} \qquad (22)$$

$$y = \frac{d \tan \gamma}{\tan \gamma - \tan \lambda} \qquad (23)$$

In other words, the position coordinate of the first end point M in the coordinate system XOY is ( $\frac{d \tan \gamma \tan \lambda}{\tan \gamma - \tan \lambda}$ , $\frac{d \tan \gamma}{\tan \gamma - \tan \lambda}$ ). By using the same logical-calculation method, the position coordinate of the second end point N of the face of the patient can be obtained, which is not discussed further in the present embodiment.

Optionally, in the present embodiment, the step of, according to the positions of the information collecting points, determining the third size of the third to-be-measured human face in the fourth to-be-measured human-face image includes but is not limited to: acquiring a value in pixel of the preset connecting line and a value in pixel of the second to-be-measured human face in the fourth to-be-measured human-face image; according to the plane-coordinate position of the first end point and the plane-coordinate position of the second end point, determining a size of the preset connecting line; and according to the size of the preset connecting line, the value in pixel of the preset connecting line and the value in pixel of the third to-be-measured human face, determining the third size.

Particularly, in an example, if the coordinate of the second end point N is set to be (*m,n*), then the position coordinate of the second end point N in the coordinate system XOY may be obtained, and in turn it may be obtained that the distance between the first end point M and the second end point N of the preset connecting line in the face of the to-be-measured target is:

$$|MN| = \sqrt{(x-m)^2 + (y-n)^2} \qquad (24)$$

In other words, by using the above-described double-lens processing system, the distance between any two information collecting points in the face of the to-be-measured target may be obtained, to determine the third size of the third to-be-measured human face, which is used to instruct the to-be-measured target to select a suitable face mask, or used to manufacture a suitable face mask.

Optionally, in the present embodiment, the third image collecting device includes at least three cameras, and the at least three cameras are located in a same plane; the fourth to-be-measured human-face image includes a plurality of information collecting points, and the information collecting points are allocated into the fourth to-be-measured human-face image according to a preset density threshold; and the step of acquiring the at least two incident angles of inclination corresponding to the information collecting points in the fourth to-be-measured human-face image when the diffuse reflection irradiates into the at least two cameras includes but is not limited to: acquiring at least three incident angles of inclination corresponding to the information collecting points.

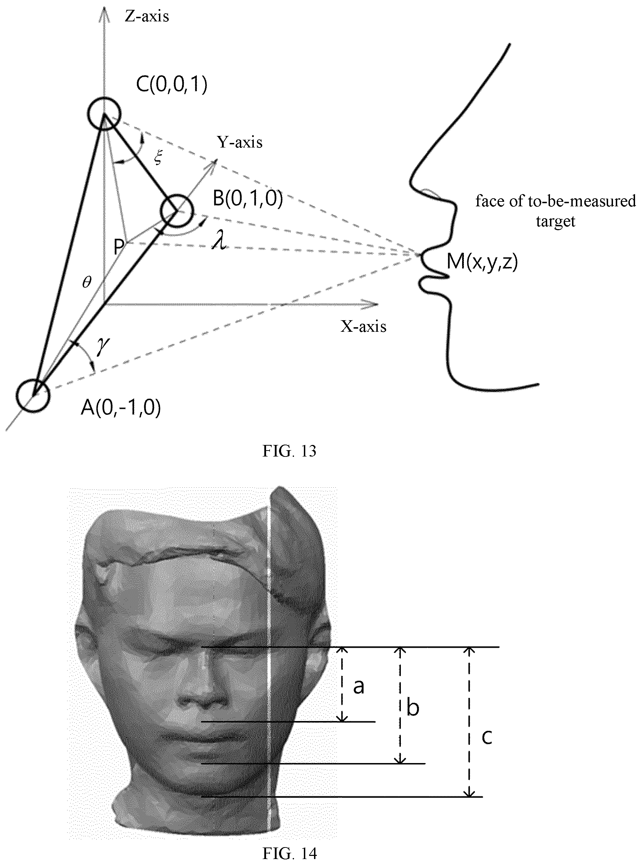

Particularly, referring to FIG. 13, FIG. 13 shows a schematic diagram of a method for determining a human-face size based on a three-camera image collecting device according to the present disclosure. The third image collecting device includes a camera A, a camera B and a camera C, and has the function of data processing. The plane where the camera A, the camera B and the camera C are located is marked by using the Greek letter θ. The camera A, the camera B and the camera C can capture light rays, and sense the incident angles of inclination of the light rays relative to the plane θ. One information collecting point M is randomly taken in the face of the to-be-measured target, and the light rays that it diffusively reflects individually enter the camera A, the camera B and the camera C. The camera A captures the light ray MA from the information collecting point M, and calculates to obtain its incident angle of inclination $\gamma$ relative to the plane θ. The camera B captures the light ray MB from the information collecting point M, and calculates to obtain its incident angle of inclination $\lambda$ relative to the plane θ. The camera C captures the light ray MC from the information collecting point M, and calculates to obtain its incident angle of inclination $\zeta$ relative to the plane θ.

Optionally, in the present embodiment, the step of, according to the at least two incident angles of inclination and the camera distance between the at least two cameras, determining the positions of the information collecting points includes but is not limited to: acquiring at least three distances between each of the information collecting points and the at least three cameras; according to a plane where the at least three cameras are located, establishing a space coordinate system; and based on the space coordinate system, according to camera distances between the at least three cameras and the at least three incident angles, determining space coordinate positions of the information collecting points.

Particularly, still taking the example in FIG. 13 as an example for the description, in order to conveniently determine the position of the information collecting point M, the present embodiment provides the following preferable logical-calculation method. A space rectangular coordinate system is established, and it is defined that the coordinate positions of the camera A, the camera B and the camera C are A (0,-1,0), B (0,1,0) and C (0,0,1) respectively. It is set that the coordinate of the information collecting point M is (x,y,z), it may be easily known that the projection point of the information collecting point M in the plane θ is P (0,y,z).

Accordingly, the following vector relations can be obtained:

$$AP = (0, y+1, z); AM = (x, y+1, z); \qquad (25)$$

$$BP = (0, y-1, z); BM = (x, y-1, z); \qquad (26)$$

$$CP = (0, y, z-1); CM = (x, y, z-1) \qquad (27)$$

$$\cos \gamma = \frac{AP \cdot AM}{|AP| \cdot |AM|} = \frac{(y+1)^2 + z^2}{\sqrt{(y+1)^2 + z^2} \cdot \sqrt{x^2 + (y+1)^2 + z^2}} \qquad (28)$$

$$\cos \lambda = \frac{BP \cdot BM}{|BP| \cdot |BM|} = \frac{(y-1)^2 + z^2}{\sqrt{(y-1)^2 + z^2} \cdot \sqrt{x^2 + (y-1)^2 + z^2}} \qquad (29)$$

$$\cos \xi = \frac{CP \cdot CM}{|CP| \cdot |CM|} = \frac{y^2 + (z-1)^2}{\sqrt{y^2 + (z-1)^2} \cdot \sqrt{x^2 + y^2 + (z-1)^2}} \qquad (30)$$

By simultaneously solving the formulas (28)-(30), the values of x, y and z can be obtained, or, in other words, the space coordinate position of the information collecting point M is obtained.

Optionally, in the present embodiment, the step of, according to the positions of the information collecting points, determining the third size of the third to-be-measured human face in the fourth to-be-measured human-face image includes but is not limited to: based on the space coordinate positions corresponding to the plurality of information collecting points, establishing a human-face model of the third to-be-measured human face, to determine the third size.

Particularly, referring to FIG. 14, FIG. 14 shows a schematic diagram of a human-face model of a to-be-measured target according to an embodiment of the present disclosure. In the present embodiment, the curved surface of the face of the to-be-measured target is formed by combining a plurality of information collecting points, and, particularly, the quantity of the information collecting points may be set according to practical experience. All of the data from the information collecting points in the face of the to-be-measured target are collected and calculated by the third image collecting device of a three-lens processing system, which acquires the space coordinate positions of each of the information collecting points, which can in turn form a human-face 3D model of the face of the to-be-measured target. In particular application scenes, the human-face 3D model may be used by a computer to automatically identify particular size information, which is used to select a face mask matching with the face of the to-be-measured target. Certainly, it may also be used to, according to the 3D model of the face of the to-be-measured target, design a personalized face mask.

It should be noted that, in particular application scenes, especially regarding a to-be-measured target having a malformed face, by designing a matching face mask according to the face information of the to-be-measured target, the sealing property and the wearing comfortableness are effectively improved.

The present embodiment includes by using a third image collecting device, collecting a fourth to-be-measured human-face image of a to-be-measured target, wherein the image collecting device includes at least two cameras; acquiring at least two incident angles of inclination corresponding to information collecting points in the fourth to-be-measured human-face image; according to the at least two incident angles of inclination and a camera distance between the at least two cameras, determining positions of the information collecting points; and according to the positions of the information collecting points, determining a third size of a third to-be-measured human face in the fourth to-be-measured human-face image. That avoids manually measuring the human face by using a scale ruler, which simplifies the measuring operation. Moreover, by photographing the fourth to-be-measured human-face image by using a plurality of lenses, the third size of the third to-be-measured human face can be determined based on the camera distance between the cameras, which ensures the accuracy of the measurement on the human-face size.

The fourth method embodiment

The present embodiment further provides a method for determining a face-mask size. The method may particularly include the following steps:

Step S1: by using the method for determining a human-face size according to any one or more of the first method embodiment, the second method embodiment and the third method embodiment, obtaining a target human-face size; and

Step S2: according to a plurality of groups of preset face-mask reference values and the target human-face size, determining a face-mask model of the to-be-measured target, wherein the plurality of groups of preset face-mask reference values correspond to a plurality of face-mask models.

The target human-face size is obtained by using the methods in the first method embodiment, the second method embodiment or the third method embodiment, and the particular process of acquiring the target human-face size is not discussed further in the present embodiment.

Subsequently, in particular application scenes, the face masks of different models correspond to different human-face-size ranges, and each of the human-face-size ranges corresponds to one group of preset face-mask reference values. If the relevant parameters of the target human-face size fall within the human-face-size range corresponding to the preset face-mask reference values, then it is determined that the face-mask model corresponding to the current preset face-mask reference values is the face-mask model matching with the to-be-measured target.

Particularly, in the present embodiment, the face-mask reference values include but are not limited to the parameters such as the nose width, the first distance between the two inner canthi in the face, and the second distance between the eyebrow center and the philtrum. The particular types of the parameters of the face sites may be set according to practical experience, and are not limited in any form in the present embodiment.

Optionally, in the present embodiment, each of the groups of the face-mask reference values includes a first reference value corresponding to a first human-face site and a second reference value corresponding to a second human-face site, and the target human-face size includes a plurality of site sizes corresponding to the first face site and the second face site; and the step of, according to the plurality of groups of preset face-mask reference values and the target human-face size, determining the face-mask model of the to-be-measured target includes but is not limited to: in response to the site size corresponding to the first face site matching with the first reference value, determining the face-mask model corresponding to the first reference value.

In particular application scenes, the face sites of the human face are classified according to the gas-tightness priorities, wherein the face site that has a higher gas-tightness priority and/or a higher comfortableness weight value is classified as the first face site, and the face site that has a lower gas-tightness priority and/or a lower comfortableness weight value is classified as the second face site. If the gas-tightness priority is higher, the face mask has a better gas tightness, and if the comfortableness weight value is higher, the user has a higher comfortableness.

In the present embodiment, because all of the reference values corresponding to the different sites are different, the face sites in the target human face might not completely match with the reference values of one face-mask model. In this case, the corresponding face-mask model is determined according to the first reference value matching with the first face site, to ensure the gas tightness and the comfortableness of the face mask.

Optionally, in the present embodiment, the step of, according to the plurality of groups of preset face-mask reference values and the target human-face size, determining the face-mask model of the to-be-measured target includes but is not limited to: in response to the plurality of site sizes in the target human-face size completely matching with the plurality of reference values in the face-mask reference values, selecting the face-mask model corresponding to the face-mask reference values; or in response to the plurality of site sizes in the target human-face size partially matching with the plurality of reference values in the face-mask reference values, when a quantity of the matched reference values is greater than or equal to a preset quantity threshold, selecting the face-mask model corresponding to the face-mask reference values.

Particularly, in the present embodiment, the step of, according to the plurality of groups of preset face-mask reference values and the target human-face size, determining the face-mask model of the to-be-measured target includes matching a plurality of site sizes in the target human-face size and a plurality of site reference values in the face-mask reference values; in response to the plurality of site sizes in the target human-face size completely matching with the plurality of reference values in the face-mask reference values, selecting the face-mask model corresponding to the face-mask reference values; and in response to the plurality of site sizes in the target human-face size partially matching with the plurality of reference values in the face-mask reference values, when a quantity of the matched reference values is greater than or equal to a preset quantity threshold, selecting the face-mask model corresponding to the face-mask reference values.

In an example, the nose width, the first distance between the two inner canthi in the face, and the second distance between the eyebrow center and the philtrum in the target human-face size are matched with the face-mask reference values. If all of the nose width, the first distance and the second distance match with the site reference values of a face-mask model A, then it is determined that the face-mask model matching with the target person is the model A. If the nose width matches with the site reference value corresponding to the face-mask model A, and both of the first distance and the second distance match with the site reference values of a face-mask model B, then it is determined that the face-mask model matching with the target person is the model B.

In the above embodiment, by, according to a plurality of groups of preset face-mask reference values and the target human-face size, determining a face-mask model of the to-be-measured target, the gas tightness and the comfortableness of the face mask are ensured.

The first device embodiment

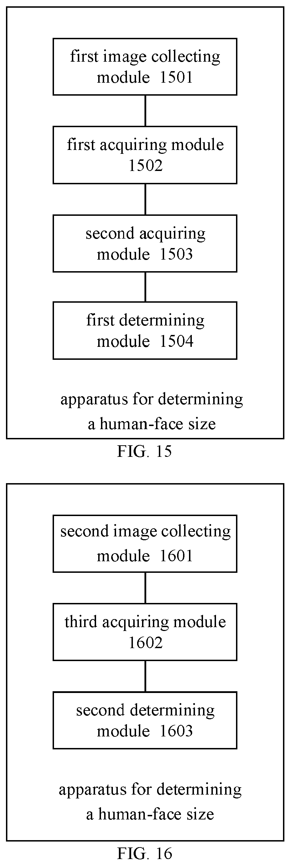

Referring to FIG. 15, FIG. 15 shows a structural block diagram of the first embodiment of the apparatus for determining a human-face size according to the present disclosure. The apparatus may particularly include:

1) a first image collecting module 1501 configured for collecting a first to-be-measured human-face image of a to-be-measured target, wherein the first to-be-measured human-face image includes a first to-be-measured human face of the to-be-measured target and a circular reference object;
2) a first acquiring module 1502 configured for acquiring a first value in pixel of the first to-be-measured human face, and a second value in pixel of the circular reference object;
3) a second acquiring module 1503 configured for, in response to a circularity of the circular reference object being greater than a preset circularity threshold, acquiring an angle of inclination of the circular reference object; and
4) a first determining module 1504 configured for, according to the first value in pixel, the second value in pixel, the angle of inclination and an actual size of the circular reference object, determining a first size of the first to-be-measured human face.

Optionally, the particular examples of the present embodiment may refer to the examples described above with respect to the first process embodiment, and are not discussed further in the present embodiment.

The second device embodiment

Referring to FIG. 16, FIG. 16 shows a structural block diagram of the second embodiment of the apparatus for determining a human-face size according to the present disclosure. The apparatus may particularly include:

1) a second image collecting module 1601 configured for, by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal;
2) a third acquiring module 1602 configured for acquiring a second value in pixel of the second to-be-measured human-face image, and a third value in pixel of the third to-be-measured human-face image; and
3) a second determining module 1603 configured for, according to an image-collection-distance difference between the second to-be-measured human-face image and the third to-be-measured human-face image, a focal length of the second image collecting device, the second value in pixel and the third value in pixel, determining a second size of a second to-be-measured human face of the to-be-measured target.

Optionally, the particular examples of the present embodiment may refer to the examples described above with respect to the second process embodiment, and are not discussed further in the present embodiment.

The third device embodiment

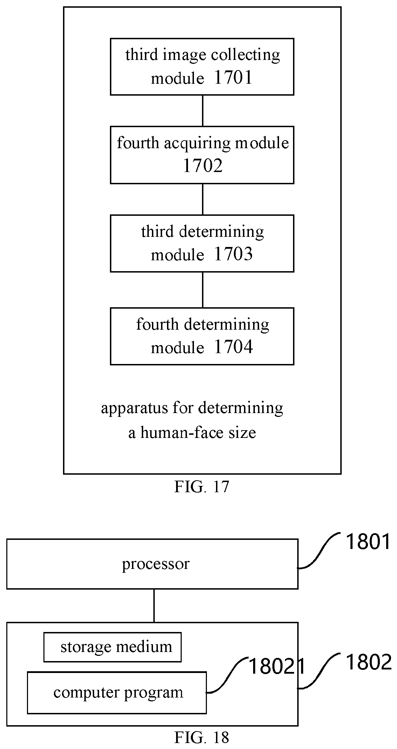

Referring to FIG. 17, FIG. 17 shows a structural block diagram of the third embodiment of the apparatus for determining a human-face size according to the present disclosure. The apparatus may particularly include:

1) a third image collecting module 1701 configured for, by using a third image collecting device, collecting a fourth to-be-measured human-face image of a to-be-measured target, wherein the third image collecting device comprises at least two cameras;
2) a fourth acquiring module 1702 configured for acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras;
3) a third determining module 1703 configured for, according to the at least two incident angles of inclination and a camera distance between the at least two cameras, determining positions of the information collecting points; and
4) a fourth determining module 1704 configured for, according to the positions of the information collecting points, determining a third size of a third to-be-measured human face in the fourth to-be-measured human-face image.

Optionally, the particular examples of the present embodiment may refer to the examples described above with respect to the third process embodiment, and are not discussed further in the present embodiment.

The fourth device embodiment

The present embodiment further provides an apparatus for determining a face-mask model, wherein the apparatus includes:

1) an acquiring unit configured for, by using the apparatus for determining a human-face size according to any one of the first device embodiment, the second device embodiment and the third device embodiment, obtaining a target human-face size; and
2) a determining unit configured for, according to a plurality of groups of preset face-mask reference values and the target human-face size, determining a face-mask model of the to-be-measured target, wherein the plurality of groups of preset face-mask reference values correspond to a plurality of face-mask models.

Optionally, the particular examples of the present embodiment may refer to the examples described above with respect to the fourth process embodiment, and are not discussed further in the present embodiment.
Regarding the device embodiments, because they are substantially similar to the process embodiments, they are described simply, and the related parts may refer to the description on the process embodiments.
The embodiments of the description are described in the mode of progression, each of the embodiments emphatically describes the differences from the other embodiments, and the same or similar parts of the embodiments may refer to each other.
The particular modes of the operations performed by the modules of the apparatus according to the above embodiments have already been described in detail in the embodiments of the method, and will not be explained and described in detail herein.
An embodiment of the present application further provides an electronic device. Referring to FIG. 18, the electronic device comprises a processor 1801, a memory 1802 and a computer program 18021 that is stored in the memory and is executable in the processor, wherein the processor, when executing the computer program, implements the method for determining a human-face size according to the above embodiments.
An embodiment of the present application further provides a readable storage medium, wherein when an instruction in the storage medium is executed by a processor of an electronic device, the electronic device is able to implement the method for determining a human-face size according to the above embodiments.
Regarding the device embodiments, because they are substantially similar to the process embodiments, they are described simply, and the related parts may refer to the description on the process embodiments.
The algorithms and displaying provided herein are not inherently related to any particular computer, virtual system or other devices. Various general-purpose systems may be used with the teachings disclosed herein. On the basis of the above description, the structure required to construct such a type of systems is apparent. Furthermore, the embodiments of the present application are not limited to any specific programming language. It should be understood that the contents of the embodiments of the present application described herein may be implemented by using various programming languages, and the description above for a specific language is intended to disclose the most preferable embodiments of the embodiments of the present application.
Each component embodiment of the embodiments of the present application may be implemented by hardware, or by software modules that are operated in one or more processors, or by a combination thereof. A person skilled in the art should understand that some or all of the functions of some or all of the components of the device according to the embodiments of the present application may be implemented by using a microprocessor or a digital signal processor (DSP) in practice. The embodiments of the present application may also be implemented as apparatus or device programs for implementing part of or the whole of the method described herein. Such programs for implementing the embodiments of the present application may be stored in a computer-readable medium, or may be in the form of one or more signals. Such signals may be downloaded from an Internet website, or provided on a carrier signal, or provided in any other forms.

Claims

1. A method for determining a human-face size, **characterized in that** the method comprises:

collecting (S301) a first to-be-measured human-face image of a to-be-measured target, wherein the first to-be-measured human-face image comprises a first to-be-measured human face of the to-be-measured target and a circular reference object;
acquiring (S302) a first value in pixels of the first to-be-measured human face, and a second value in pixels of the circular reference object;

in response to a circularity of the circular reference object being greater than a preset circularity threshold, acquiring (S303) an angle of inclination of the circular reference object; and

according to the first value in pixels, the second value in pixels, the angle of inclination and an actual size of the circular reference object, determining (S304) a first size of the first to-be-measured human face;

wherein the circularity refers to the difference between the radii of two concentric circles that include the same actual cross-sectional contour and have the lowest radius difference.

2. The method according to claim 1, **characterized in that** the method further comprises:

in response to the circularity being less than or equal to the preset circularity threshold, according to the first value in pixels, the second value in pixels and the actual size, determining the first size.

3. The method according to claim 2, **characterized in that** the step of, in response to the circularity of the circular reference object being greater than the preset circularity threshold, acquiring the angle of inclination of the circular reference object comprises:

acquiring a standard major axis of the circular reference object, wherein the standard major axis refers to, in response to the circularity being greater than the preset circularity threshold, a longest diameter in the circular reference object;

acquiring a first major axis in a first direction and a second major axis in a second direction in the circular reference object, wherein the first direction refers to a straight line where a connecting line between centers of two eyes in the first to-be-measured human-face image is located, and the second direction refers to a straight line where a connecting line between an eyebrow center and a nasal tip in the first to-be-measured human-face image is located;

according to the standard major axis and the first major axis, determining a horizontal angle of inclination of the circular reference object; and

according to the standard major axis and the second major axis, determining a vertical angle of inclination of the circular reference object.

4. The method according to claim 3, **characterized in that** the step of, according to the first value in pixels, the second value in pixels, the angle of inclination and the actual size of the circular reference object, determining the first size of the first to-be-measured human face comprises:

according to the first value in pixels, the second value in pixels, the horizontal angle of inclination, the vertical angle of inclination and the actual size, determining the first size.

5. The method according to claim 1, **characterized in that** the circular reference object comprises an iris in the first to-be-measured human-face image.

6. A method for determining a face-mask model, **characterized in that** the method comprises:

by using the method for determining a human-face size according to any one of claims 1-5, obtaining a target human-face size; and

according to a plurality of groups of preset face-mask reference values and the target human-face size, determining a face-mask model of the to-be-measured target, wherein the plurality of groups of preset face-mask reference values correspond to a plurality of face-mask models.

7. The method according to claim 6, **characterized in that** each of the groups of the face-mask reference values includes a first reference value corresponding to a first human-face site and a second reference value corresponding to a second human-face site, and the target human-face size comprises a plurality of site sizes corresponding to the first face site and the second face site; and

the step of, according to the plurality of groups of preset face-mask reference values and the target human-face size, determining the face-mask model of the to-be-measured target comprises:

in response to the site size corresponding to the first face site matching with the first reference value, determining the face-mask model corresponding to the first reference value.

8. The method according to claim 7, **characterized in that** the step of, according to the plurality of groups of preset face-mask reference values and the target human-face size, determining the face-mask model of the to-be-measured target comprises:

in response to the plurality of site sizes in the target human-face size completely matching with a plurality of site reference values in the face-mask reference values, selecting the face-mask model corresponding to the face-mask reference values; or

in response to the plurality of site sizes in the target human-face size partially matching with the plurality of site reference values in the face-mask reference values, when a quantity of the matched site reference values is greater than or equal to a preset quantity threshold, selecting the face-mask model corresponding to the face-mask reference values.

9. An electronic device, **characterized in that** the electronic device comprises:

a memory storing a computer-readable code; and
one or more processors, wherein when the computer-readable code is executed by the one or more processors, a computing and processing device implements the method for determining a human-face size according to any one of claims 1-5, or the method for determining a face-mask model according to any one of claims 6-8.

10. A computer program, **characterized in that** the computer program comprises a computer-readable code, and when the computer-readable code is executed in an electronic device, the computer-readable code causes the electronic device to implement the method for determining a human-face size according to any one of claims 1-5, or the method for determining a face-mask model according to any one of claims 6-8.

11. A computer-readable storage medium, **characterized in that** the computer-readable storage medium stores the computer program according to claim 10.

**Patentansprüche**

1. Verfahren zur Bestimmung der Größe eines menschlichen Gesichts, **dadurch gekennzeichnet, dass** das Verfahren umfasst:

Erfassen (S301) eines ersten zu messenden Gesichtsbildes eines zu messenden Ziels, wobei das erste zu messende Gesichtsbild ein erstes zu messendes menschliches Gesicht des zu messenden Ziels und ein kreisförmiges Referenzobjekt aufweist;
Erfassen (S302) eines ersten Wertes in Pixeln des ersten zu messenden menschlichen Gesichts und eines zweiten Wertes in Pixeln des kreisförmigen Referenzobjekts;
Erfassen (S303) eines Neigungswinkels des kreisförmigen Referenzobjekts, wenn dessen Rundheit größer ist als ein voreingestellter Rundheitsschwellenwert; und
Bestimmen (S304) einer ersten Größe des ersten zu messenden menschlichen Gesichts anhand des ersten Pixelwerts, des zweiten Pixelwerts, des Neigungswinkels und einer tatsächlichen Größe des kreisförmigen Referenzobjekts;
wobei sich die Rundheit auf die Differenz zwischen den Radien zweier konzentrischer Kreise bezieht, die dieselbe tatsächliche Querschnittskontur enthalten und die geringste Radiusdifferenz aufweisen.

2. Verfahren nach Anspruch 1, **dadurch gekennzeichnet, dass** das Verfahren ferner umfasst:
als Reaktion darauf, dass die Rundheit kleiner oder gleich dem voreingestellten Rundheitsschwellenwert ist, Bestimmen, unter Berücksichtigung des ersten Wertes in Pixeln, des zweiten Wertes in Pixeln und der tatsächlichen Größe, der ersten Größe.

3. Verfahren nach Anspruch 2, **dadurch gekennzeichnet, dass** der Schritt,
als Reaktion darauf, dass die Rundheit des kreisförmigen Referenzobjekts größer als der voreingestellte Rundheitsschwellenwert ist, Erfassen des Neigungswinkels des kreisförmigen Referenzobjekts, umfasst:

Erfassen einer Standard-Hauptachse des kreisförmigen Referenzobjekts, wobei sich die Standard-Hauptachse, als Reaktion darauf, dass die Rundheit größer als der voreingestellte Rundheitsschwellenwert ist, auf einen längsten Durchmesser in dem kreisförmigen Referenzobjekt bezieht;
Erfassen einer ersten Hauptachse in einer ersten Richtung und einer zweiten Hauptachse in einer zweiten Richtung in dem kreisförmigen Referenzobjekt, wobei sich die erste Richtung auf eine Gerade bezieht, auf der sich eine Verbindungslinie zwischen den Mittelpunkten zweier Augen im ersten zu messenden menschlichen Gesichtsbild befindet, und die zweite Richtung sich auf eine Gerade bezieht, auf der sich eine Verbindungslinie zwischen einer Augenbrauenmitte und einer Nasenspitze in dem ersten zu messenden Gesichtsbild befindet; Bestimmen eines horizontalen Neigungswinkels des kreisförmigen Referenzobjekts anhand der Standard-Hauptachse und der ersten Hauptachse; und Bestimmen eines vertikalen Neigungswinkels des kreisförmigen Referenzobjekts anhand der Standard-Hauptachse und der zweiten Hauptachse.

4. Verfahren nach Anspruch 3, **dadurch gekennzeichnet, dass** der Schritt des Bestimmens der ersten Größe des ersten zu messenden menschlichen Gesichts anhand des ersten Wertes in Pixeln, des zweiten Wertes in Pixeln, des Neigungswinkels und der tatsächlichen Größe des kreisförmigen Referenzobjekts umfasst:

   Bestimmen der ersten Größe anhand des ersten Wertes in Pixeln, des zweiten Wertes in Pixeln, des horizontalen Neigungswinkels, des vertikalen Neigungswinkels und der tatsächlichen Größe.

5. Verfahren nach Anspruch 1, **dadurch gekennzeichnet, dass** das kreisförmige Referenzobjekt eine Iris in dem ersten zu messenden menschlichen Gesichtsbild enthält.

6. Verfahren zum Bestimmen eines Gesichtsmaskenmodells, **dadurch gekennzeichnet, dass** das Verfahren umfasst:

   Ermitteln einer Zielgröße des menschlichen Gesichts unter Verwendung des Verfahrens zum Bestimmen einer menschlichen Gesichtsgröße gemäß einem der Ansprüche 1 bis 5; und
   auf der Grundlage einer Vielzahl von Gruppen voreingestellter Gesichtsmasken-Referenzwerte und der Zielgröße des menschlichen Gesichts, Bestimmen eines Gesichtsmaskenmodells des zu messenden Ziels, wobei die Vielzahl von Gruppen voreingestellter Gesichtsmasken-Referenzwerte einer Vielzahl von Gesichtsmaskenmodellen entspricht.

7. Verfahren nach Anspruch 6, **dadurch gekennzeichnet, dass** jede der Gruppen der Gesichtsmasken-Referenzwerte einen ersten Referenzwert, der einer ersten Stelle des menschlichen Gesichts entspricht, und einen zweiten Referenzwert, der einer zweiten Stelle des menschlichen Gesichts entspricht, enthält, und die Zielgröße des menschlichen Gesichts eine Vielzahl von Stellengrößen aufweist, die der ersten Gesichtsstelle und der zweiten Gesichtsstelle entsprechen; und
   der Schritt des Bestimmens des Gesichtsmaskenmodells des zu messenden Ziels gemäß der Vielzahl von Gruppen voreingestellter Gesichtsmasken-Referenzwerte und der Zielgröße des menschlichen Gesichts umfasst:
   als Reaktion darauf, dass die der ersten Gesichtstelle entsprechende Stellengröße mit dem ersten Referenzwert übereinstimmt, Bestimmen des dem ersten Referenzwert entsprechenden Gesichtsmaskenmodells.

8. Verfahren nach Anspruch 7, **dadurch gekennzeichnet, dass** der Schritt des Bestimmens des Gesichtsmaskenmodells des zu messenden Ziels entsprechend der Vielzahl von Gruppen voreingestellter Gesichtsmasken-Referenzwerte und der Zielgröße des menschlichen Gesichts umfasst:

   als Reaktion darauf, dass die Vielzahl von Stellengrößen in der Zielgröße des menschlichen Gesichts vollständig mit einer Vielzahl von Stellenreferenzwerten in den Gesichtsmasken-Referenzwerten übereinstimmt, Auswählen des den Gesichtsmasken-Referenzwerten entsprechenden Gesichtsmaskenmodells; oder
   als Reaktion darauf, dass die Vielzahl von Stellengrößen in der Zielgröße des menschlichen Gesichts teilweise mit der Vielzahl von Stellenreferenzwerten in den Gesichtsmasken-Referenzwerten übereinstimmt, wenn eine Anzahl der übereinstimmenden Stellenreferenzwerte größer oder gleich einem voreingestellten Schwellenwert ist, Auswählen des den Gesichtsmasken-Referenzwerten entsprechenden Gesichtsmaskenmodells.

9. Elektronisches Gerät, **dadurch gekennzeichnet, dass** das elektronische Gerät aufweist:

   einen Speicher, der einen computerlesbaren Code speichert; und
   einen oder mehrere Prozessoren, wobei, wenn der computerlesbare Code von dem einen oder den mehreren Prozessoren ausgeführt wird, eine Rechen- und Verarbeitungsvorrichtung das Verfahren zur Bestimmung einer menschlichen Gesichtsgröße nach einem der Ansprüche 1 bis 5 oder das Verfahren zur Bestimmung eines Gesichtsmaskenmodells nach einem der Ansprüche 6 bis 8 implementiert.

10. Computerprogramm, **dadurch gekennzeichnet, dass** das Computerprogramm einen computerlesbaren Code enthält und dass, wenn der computerlesbare Code in einer elektronischen Vorrichtung ausgeführt wird, der computerlesbare Code die elektronische Vorrichtung veranlasst, das Verfahren zur Bestimmung einer menschlichen Gesichtsgröße nach einem der Ansprüche 1 bis 5 oder das Verfahren zur Bestimmung eines Gesichtsmaskenmodells nach einem der Ansprüche 6 bis 8 zu implementieren.

**11.** Computerlesbares Speichermedium, **dadurch gekennzeichnet, dass** das computerlesbare Speichermedium das Computerprogramm nach Anspruch 10 speichert.

**Revendications**

**1.** Procédé pour déterminer une taille de visage humain, **caractérisé en ce que** le procédé comprend :

la collecte (S301) d'une première image de visage humain à mesurer d'une cible à mesurer, dans lequel la première image de visage humain à mesurer comprend un premier visage humain à mesurer de la cible à mesurer et un objet de référence circulaire ;
l'acquisition (S302) d'une première valeur en pixels du premier visage humain à mesurer, et d'une seconde valeur en pixels de l'objet de référence circulaire ;
en réponse au fait qu'une circularité de l'objet de référence circulaire est supérieure à un seuil de circularité prédéfini, l'acquisition (S303) d'un angle d'inclinaison de l'objet de référence circulaire ; et
selon la première valeur en pixels, la seconde valeur en pixels, l'angle d'inclinaison et une taille réelle de l'objet de référence circulaire, la détermination (S304) d'une première taille du premier visage humain à mesurer ;
dans lequel la circularité fait référence à la différence entre les rayons de deux cercles concentriques qui contiennent le même contour en coupe transversale réelle et qui ont la différence de rayon la plus faible.

**2.** Procédé selon la revendication 1, **caractérisé en ce que** le procédé comprend en outre :
en réponse au fait que la circularité est inférieure ou égale au seuil de circularité prédéfini, selon la première valeur en pixels, la seconde valeur en pixels et la taille réelle, la détermination de la première taille.

**3.** Procédé selon la revendication 2, **caractérisé en ce que**, en réponse au fait que la circularité de l'objet de référence circulaire est supérieure au seuil de circularité prédéfini, l'étape de l'acquisition de l'angle d'inclinaison de l'objet de référence circulaire comprend :

l'acquisition d'un axe majeur standard de l'objet de référence circulaire, dans lequel l'axe majeur standard fait référence, en réponse au fait que la circularité est supérieure au seuil de circularité prédéfini, à un diamètre le plus long dans l'objet de référence circulaire ;
l'acquisition d'un premier axe majeur dans une première direction et un second axe majeur dans une seconde direction dans l'objet de référence circulaire, dans lequel la première direction fait référence à une ligne droite où se trouve une ligne de connexion entre des centres de deux yeux dans la première image de visage humain à mesurer,
et la seconde direction fait référence à une ligne droite où se trouve une ligne de connexion entre un centre de sourcil et une pointe nasale dans la première image de visage humain à mesurer ;
selon l'axe majeur standard et le premier axe majeur, la détermination d'un angle d'inclinaison horizontal de l'objet de référence circulaire ; et
selon l'axe majeur standard et le second axe majeur, la détermination d'un angle d'inclinaison vertical de l'objet de référence circulaire.

**4.** Procédé selon la revendication 3, **caractérisé en ce que**, selon la première valeur en pixels, la seconde valeur en pixels, l'angle d'inclinaison et la taille réelle de l'objet de référence circulaire, l'étape de la détermination de la première taille du premier visage humain à mesurer comprend :
selon la première valeur en pixels, la seconde valeur en pixels, l'angle d'inclinaison horizontal, l'angle d'inclinaison vertical et la taille réelle, la détermination de la première taille.

**5.** Procédé selon la revendication 1, **caractérisé en ce que** l'objet de référence circulaire comprend un iris dans la première image de visage humain à mesurer.

**6.** Procédé pour déterminer un modèle de masque facial, **caractérisé en ce que** le procédé comprend :

en utilisant le procédé pour déterminer une taille de visage humain selon l'une quelconque des revendications 1 à 5, l'obtention d'une taille de visage humain cible ; et
selon une pluralité de groupes de valeurs de référence prédéfinies de masque facial et la taille de visage humain cible, la détermination d'un modèle masque facial de la cible à mesurer, dans lequel la pluralité des groupes de valeurs de référence prédéfinies de masque facial correspond à une pluralité de modèles de masque facial.

7. Procédé selon la revendication 6, **caractérisé en ce que** chacun des groupes des valeurs de référence de masque facial comporte une première valeur de référence correspondant à un premier site de visage humain et une seconde valeur de référence correspondant à un second site de visage humain, et la taille de visage humain cible comprend une pluralité de tailles de site correspondant au premier site de visage et au second site de visage ; et
selon la pluralité de groupes de valeurs de référence prédéfinies de masque facial et la taille cible de visage humain, l'étape de la détermination du modèle de masque facial de la cible à mesurer comprend :
en réponse à la taille de site correspondant au premier site de visage qui coïncide avec la première valeur de référence, la détermination du modèle de masque facial correspondant à la première valeur de référence.

8. Procédé selon la revendication 7, **caractérisé en ce que**, selon la pluralité de groupes de valeurs de référence prédéfinies de masque facial et la taille cible de visage humain, l'étape de la détermination du modèle de masque facial de la cible à mesurer comprend :

en réponse à la pluralité de tailles de site dans la taille de visage humain cible qui coïncide complètement avec une pluralité de valeurs de référence de site dans les valeurs de référence de masque facial, la sélection du modèle de masque facial correspondant aux valeurs de référence de masque facial ; ou
en réponse à la pluralité de tailles de site dans la taille cible de visage humain qui coïncide partiellement avec la pluralité de valeurs de référence de site dans les valeurs de référence de masque facial, lorsqu'une quantité des valeurs de référence de site coïncidentes est supérieure ou égale à un seuil de quantité prédéfini, la sélection du modèle de masque facial correspondant aux valeurs de référence de masque facial.

9. Dispositif électronique, **caractérisé en ce que** le dispositif électronique comprend :

une mémoire stockant un code lisible par ordinateur ; et
un ou plusieurs processeurs, dans lequel lorsque le code lisible par ordinateur est exécuté par les un ou plusieurs processeurs, un dispositif de calcul et de traitement met en œuvre le procédé pour déterminer une taille de visage humain selon l'une quelconque des revendications 1 à 5, ou le procédé pour déterminer un modèle de masque facial selon l'une quelconque des revendications 6 à 8.

10. Programme informatique, **caractérisé en ce que** le programme informatique comprend un code lisible par ordinateur, et lorsque le code lisible par ordinateur est exécuté dans un dispositif électronique, le code lisible par ordinateur amène le dispositif électronique à mettre en œuvre le procédé pour déterminer une taille de visage humain selon l'une quelconque des revendications 1 à 5, ou le procédé pour déterminer un modèle de masque facial selon l'une quelconque des revendications 6 à 8

11. Support de stockage lisible par ordinateur, **caractérisé en ce que** le support de stockage lisible par ordinateur stocke le programme informatique selon la revendication 10.

FIG. 1

FIG. 2

collecting a first to-be-measured human-face image of a to-be-measured target, wherein the first to-be-measured human-face image comprises a first to-be-measured human face of the to-be-measured target and a circular reference object

S301

acquiring a first pixel value of the first to-be-measured human face, and a second pixel value of the circular reference object

S302

in response to a circularity of the circular reference object being greater than a preset circularity threshold, acquiring an angle of inclination of the circular reference object

S303

according to the first pixel value, the second pixel value, the angle of inclination and an actual size of the circular reference object, determining a first size of the first to-be-measured human face

S304

FIG. 3

FIG. 4

FIG. 5

FIG. 6

FIG. 7

by using a second image collecting device, collecting a second to-be-measured human-face image and a third to-be-measured human-face image of a to-be-measured target, wherein an image-collection distance of the second to-be-measured human-face image and an image-collection distance of the third to-be-measured human-face image are unequal | 801

acquiring a second pixel value of the second to-be-measured human-face image, and a third pixel value of the third to-be-measured human-face image | 802

according to an image-collection-distance difference between the second to-be-measured human-face image and the third to-be-measured human-face image, a focal length of the second image collecting device, the second pixel value and the third pixel value, determining a second size of a second to-be-measured human face of the to-be-measured target | 803

FIG. 8

FIG. 9

by using a third image collecting device, collecting a fourth to-be-measured human-face image of a to-be-measured target, wherein the image collecting device comprises at least two cameras

1001

acquiring at least two incident angles of inclination corresponding to information collecting points in the fourth to-be-measured human-face image, wherein the incident angles of inclination are obtained based on the third image collecting device when diffuse reflection from the information collecting points irradiates into the at least two cameras

1002

according to the at least two incident angles of inclination and a camera distance between the at least two cameras, determining positions of the information collecting points

1003

according to the positions of the information collecting points, determining a third size of a third to-be-measured human face in the fourth to-be-measured human-face image

1004

FIG. 10

FIG. 11

FIG. 12

FIG. 13

FIG. 14

first image collecting
module  1501

first acquiring module
1502

second acquiring
module  1503

first determining
module  1504

apparatus for determining
a human-face size

FIG. 15

second image collecting
module  1601

third acquiring module
1602

second determining
module  1603

apparatus for determining
a human-face size

FIG. 16

third image collecting
module 1701

fourth acquiring module
1702

third determining
module 1703

fourth determining
module 1704

apparatus for determining
a human-face size

FIG. 17

processor 1801

storage medium

computer program 18021 1802

FIG. 18

**REFERENCES CITED IN THE DESCRIPTION**

**Patent documents cited in the description**

- CN 202110100112
- US 20180299704 A
- US 2018117272 A1